(19) Europäisches Patentamt
European Patent Office
Office européen des brevets

(11) Publication number: **0 147 152**
**B1**

(12) # EUROPEAN PATENT SPECIFICATION

(45) Date of publication of patent specification: **29.06.88**

(51) Int. Cl.$^4$: **C 07 D 519/04,** A 61 K 31/395

(21) Application number: **84308817.0**

(22) Date of filing: **17.12.84**

(54) **Improvements in or relating to novel salts of dimeric indole-dihydroindole alkaloids.**

(30) Priority: **21.12.83 US 564119**

(43) Date of publication of application:
**03.07.85 Bulletin 85/27**

(45) Publication of the grant of the patent:
**29.06.88 Bulletin 88/26**

(84) Designated Contracting States:
**AT BE CH DE FR GB IT LI LU NL SE**

(56) References cited:
**EP-A-0 009 996**
**US-A-4 203 898**

**The file contains technical information submitted after the application was filed and not included in this specification**

(73) Proprietor: **ELI LILLY AND COMPANY**
**Lilly Corporate Center**
**Indianapolis Indiana 46285 (US)**

(72) Inventor: **Rolski, Stanislaw**
**8326 E. Goldfinch Circle**
**Indianapolis Indiana 46256 (US)**
Inventor: **Pfeiffer, Ralph Robert**
**7021 North Central Avenue**
**Indianapolis Indiana 46220 (US)**

(74) Representative: **Tapping, Kenneth George et al**
**Lilly Industries Limited Patent Department**
**Erl Wood Manor**
**Windlesham Surrey, GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

A number of indole-dihydroindole alkaloids currently are being marketed or on clinical trial as anticancer agents. These alkaloids include vincaleukoblastine (vinblastine, VLB) — marketed as VELBAN, United States Patent 3,097,137; vincristine (VCR, leurocristine) — marketed as ONCOVIN, United States Patent 3,205,220; vindesine (VDS) — marketed as ELDISINE, United States Patent 4,203,898; vinzolidine, United States Patent RE 30,560; and vinepidine, United States Patent 4,143,041.

A substantial problem associated with the administration of various vinca alkaloids is finding stable, highly crystalline salt forms. For example, the sulfate salt of vindesine produced by the normal neutralization of the free base was found to be unstable over extended periods and, in addition, was difficult to handle. After considerable effort, a suitable monosulfate form was developed as described in U.S. Patent No. 4,259,242.

Vinzolidine is another indole-dihydroindole alkaloid the sulfate salt of which has been found to be unstable. In contrast to most other vinca alkaloids, vinzolidine is orally active and must be provided in solid formulations such as capsules and tablets. While an injectable product like vindesine or vincristine can be lyophilized and sealed in a dry, inert atmosphere, capsules and tablets remain exposed to air and water vapor.

Vinzolidine sulfate prepared in eight different crystalline forms using different recrystallization solvents produced salt forms of varying stability but none was found to be sufficiently stable to meet requirements for an oral dosage form.

In accordance with the invention a 1,5-naphthalenedisulfonate salt of an indole-dihydroindole alkaloid of Formula (I)

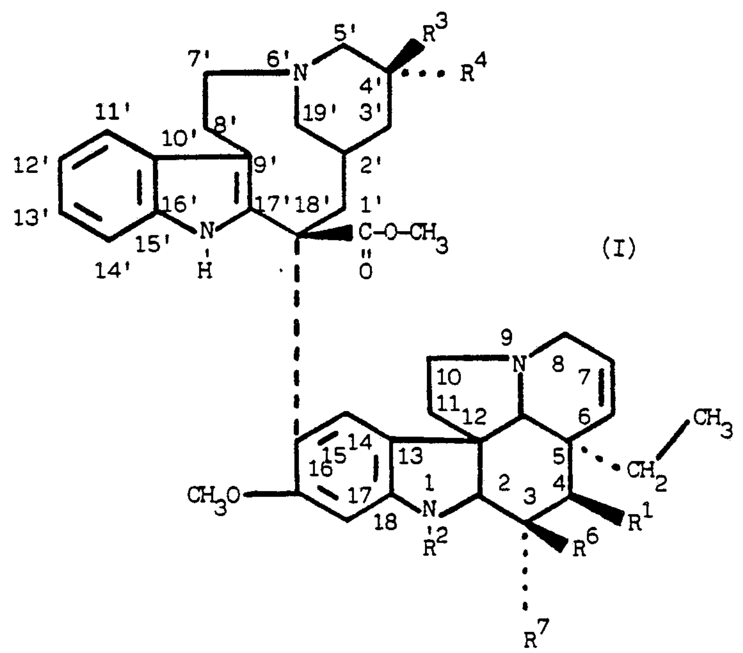

(I)

in which $R^1$ is OH or —$OCOCH_3$, $R^2$ is $CH_3$ or CHO; one of $R^3$ and $R^4$ is ethyl and the other is OH; when taken singly $R^6$ is OH and $R^7$ is $COOCH_3$ or CO—$R^9$, in which $R^9$ is $NH_2$, NH—($C_1$—$C_3$ alkyl)-X, in which X is hydrogen, chloro or bromo, or carbo-($_1$—$C_3$) alkoxy) or when $R^6$ and $R^7$ are taken together with the carbon atoms to which they are attached, they form a spiro-oxazolidenedione ring optionally substituted at $N_{3''}$ with $C_1$—$C_3$ alkyl or chloro-substituted $C_1$—$C_3$ alkyl, is a useful and stable antitumour agent.

In a preferred embodiment, there is provided a vinzolidine 1,5-napthalenedisulfonate salt which is stable for use in chemotherapy and which provides adequate blood levels of the compound upon oral administration.

Further, there are provided pharmaceutical formulations which comprise as an active ingredient, a compound of Formula (I), associated with one or more pharmaceutically-acceptable carriers or vehicles therefor.

In Formula (I) if $R^1$ is acetoxy, $R^2$ is methyl, $R^6$ is OH, $R^7$ is $COOCH_3$, $R^3$ is hydroxy and $R^4$ is ethyl, VLB (vincaleukoblastine, vinblastine) is represented; if $R^1$ is acetoxy, $R^2$ is formyl, $R^6$ is OH, $R^7$ is $COOCH_3$, $R^3$ is hydroxy and $R^4$ is ethyl, vincristine (leurocristine, VCR) is represented; if $R^1$ is acetoxy, $R^2$ is methyl, $R^6$ is OH, $R^7$ is $COOCH_3$, $R^3$ is ethyl and $R^4$ is hydroxy, leurosidine (vinrosidine) is represented; when $R^6$ and $R^7$, together with the carbon to which they are attached, form an oxazolidindione ring with a chloroethyl group at $N_{3''}$, $R^2$ is $CH_3$, $R^1$ is acetoxy, $R^3$ is OH and $R^4$ is ethyl, vinzolidine is represented; and when $R^2$ is $CH_3$, $R^3$, $R^6$ and $R^1$ are OH, $R^4$ is ethyl, $R^7$ is $CONH_2$, vindesine is represented.

To further illustrate the invention, the following non-limiting examples are provided.

## Example 1

Vinzolidine 1,5-naphthalenedisulfonate

To a 1% (w/v) solution of vinzolidine free base (95+ % purity) in methanol is added dropwise with stirring an equimolar amount (1 mole per mole of free base) of a 2% methanolic solution of 1,5-napthalenedisulfonic acid. The resulting solution was allowed to stand at ambient temperature for about two hours, during which crystallization of the vinzolidine 1,5-naphthalenedisulfonate salt begins. The crystallizing solution is kept at about 0°C. overnight and then is filtered. The filter cake is washed with a small amount of methanol and then dried in vacuo overnight at ambient temperature. The salt then is allowed to stand in air for about two hours. The yield of vinzolidine 1,5-naphthalenedisulfonate is 95—97%.

## Example 2

Alternate Preparation of Vinzolidine 1,5-Napthalene Disulfonate

To a 1% solution of vinzolidine free base in anhydrous ethanol, an equimolar amount of 1.0N sulfuric acid is added in dropwise fashion with stirring [1.0 g. of free base (~93% purity) requires ~ 2 ml. of 1.0N sulfuric acid]. Stirring is continued until the solution becomes opalescent. The solution is allowed to stand at room temperature for two additional hours followed by 2-3 hours at about 0°C. The precipitate is filtered on a sintered glass funnel, washed with a small amount of anhydrous ethanol and dried in vacuo at 45° producing a yield of about 85%. Additional salt (*ca.* 10%) can be obtained from the filtrate.

This sulfate salt contains about 9% of water (possibly in the form of pentahydrate) from which a sharp x-ray pattern was obtained.

A 10% aqueous solution of 1,5-naphthalenedisulfonic acid disodium salt is added dropwise with stirring to a 2% aqueous solution of the vinzolidine sulfate prepared above. A 10% excess of 1,5-naphthalenedisulfonic acid disodium salt is used (i.e., 1.1 mole of acid per mole of base). After holding the mixture at about 0°C. overnight, the precipitate is filtered on a sintered glass funnel, washed with a small amount of water, and dried in vacuo overnight at room temperature. An amorphous material, containing about 7% $H_2O$, is obtained in greater than 80% yield.

The amorphous material is added gradually to methanol with stirring (1 g. per 100 ml. of methanol). After the addition is complete, the suspension is stored in the refrigerator overnight. Subsequent handling is the same as in Example 1. The salt crystallizes as a methanolate (Form I) by both methods and contains three molecules of methanol per molecule of salt.

Vacuum drying removes the methanol but equilibration of the desired salt in air gives a hydrate (Form II); Karl Fisher ~ 8% water. Form II dissolves in water at 25°C. to the extent of about 150 mcg./ml.

A third crystalline form, Form III, is produced by recrystallizing the salt in boiling methanol and then drying the methanolate in air, which procedure yields a different hydrate containing about 6% water. The x-ray diffraction patterns of these three forms follow. The patterns were obtained using an x-ray tube with a copper target and a nickel filter.

FORM I

| d | $I/I_1$ | d | $I/I_1$ | d | $I/I_1$ |
|---|---|---|---|---|---|
| 12.15 | .80 | 4.57 | .36 | 2.89 | .04 |
| 10.88 | .16 | 4.42 | .44 | 2.77 | .04 |
| 9.80 | 1.00 | 4.17 | .40 | 2.67 | .04 |
| 8.14 | .16 | 3.97 | .32 | 2.54 | .04 |
| 7.58 | .28 | 3.76 | .64 | 2.46 | .04 |
| 7.24 | .20 | 3.56 | .40 | 2.40 | .04 |
| 6.52 | .64 | 3.34 | .44 | 2.28 | .04 |
| 6.18 | .16 | 3.26 | .24 | 2.22 | .04 |
| 5.90 | .04 | 3.08 | .04 | 2.15 | .04 |
| 5.55 | .16 | 2.95 | .04 | 2.08 | .06 |
| 4.89 | .44 | | | | |

FORM II

| d | I/I$_1$ | d | I/I$_1$ | d | I/I$_1$ |
|---|---|---|---|---|---|
| 12.65 | .14 | 4.36 | .28 | 2.91 | .03 |
| 10.54 | .48 | 4.16 | .14 | 2.79 | .03 |
| 9.47 | 1.00 | 4.07 | .14 | 2.68 | .03 |
| 7.98 | .07 | 3.92 | .07 | 2.60 | .02 |
| 7.32 | .17 | 3.78 | .14 | 2.49 | .03 |
| 7.15 | .59 | 3.57 | .07 | 2.38 | .02 |
| 6.69 | .21 | 3.47 | .28 | 2.32 | .02 |
| 6.11 | .17 | 3.34 | .17 | 2.25 | .03 |
| 5.44 | .41 | 3.23 | .07 | 2.22 | .02 |
| 5.14 | .31 | 3.13 | .07 | 2.17 | .02 |
| 4.71 | .52 | 3.03 | .03 | 2.12 | .02 |
| 4.50 | .10 | | | | |

FORM III

| d | I/I$_1$ | d | I/I$_1$ | d | I/I$_1$ |
|---|---|---|---|---|---|
| 10.85 | .55 | 5.40 | .20 | 3.42 | .15 |
| 10.16 | .05 | 5.07 | .10 | 3.30 | .05 |
| 9.26 | 1.00 | 4.75 | .40 | 3.15 | .08 |
| 7.41 | .05 | 4.33 | .30 | 3.02 | .05 |
| 6.76 | .55 | 4.20 | .15 | 2.92 | .08 |
| 6.28 | .80 | 4.00 | .20 | 2.82 | .05 |
| 5.68 | .20 | 3.75 | .05 | 2.69 | .05 |

1,5-Naphthalenedisulfonate salts were also prepared from vinblastine, vinepidine, vincristine and vindesine. The salts were stable, white solids sparingly soluble in water.

Orally active vinzolidine 1,5-naphthalenedisulfonate, (vinzolidine napadisilate) is of particular interest because, as discussed below, it is bioequivalent to vinzolidine sulfate.

This is important because dosage forms suitable for oral administration have more stringent requirements than do parenteral dosage forms; i.e., the drug must be stable when mixed with pharmaceutically-acceptable excipients customarily used in preparing solid dosage forms such as capsules or tablets. Any such modification of drug form must not substantially increase toxicity nor decrease oral efficacy.

In addition, bulk vinzolidine 1,5-naphthalenedisulfonate remained virtually unchanged at one year at the following storage temperatures — 5°C.; 25°C.; 40°C. In addition, the bulk material was stable for one year at 40°C. and 75% relative humidity. Vinzolidine 1,5-naphthalenedisulfonate-containing capsules had a potency of 94.3% of the initial potency after storage for one year at 40°C. and 75% relative humidity. Under the same storage conditions, vinzolidine sulfate is less stable.

Further, the oral $LD_{50}$ in mice of vinzolidine 1,5-naphthalenedisulfonate is 68.7 mg./kg. compared to 37.7 mg./kg. for vinzolidine sulfate.

The 1,5-naphthalenedisulfonate salts shows substantial antitumour activity against transplanted tumors in mice. For example, using the 1,5-naphthalenedisulfonate salt, in the 6C3HED lymphosarcoma screen, a single oral dose of 11 mg./kg. (in either acacia or emulphor suspension) gives about a 50% inhibition of tumor growth and single doses of 22.5 mg./kg. give 95—100% inhibition.

Finally, vinzolidine 1,5-naphthalenedisulfonate is well-absorbed when administered by the oral route. For example, when each of six monkeys was given equivalent amounts of free base in a single capsule containing vinzolidine sulfate or vinzolidine 1,5-naphthalenedisulfonate, comparable blood levels of vinzolidine were obtained. Table 1 which follows gives pooled mean serum concentrations of the naphthalenedisulfonate in mg./ml. for the six monkeys at given time intervals with the standard deviation.

TABLE 1

| Time in Hours | Mean Serum Concentration Mg./Ml. ± SD |
|---|---|
| 1/12 | 0 |
| 1/2 | 0.46 ± 0.28 |
| 1 | 29.38 ± 7.80 |
| 4 | 19.53 ± 3.25 |
| 8 | 5.32 ± 0.93 |
| 24 | 0.66 ± 0.28 |
| 48 | 0.05 ± 0.04 |
| 72 | 0 |
| 96 | 0 |

Oral formulations of vinzolidine 1,5-naphthalenedisulfonate may include telescoping gelatin capsules, tablets, suspensions and the like such that each capsule, tablet or unit of suspension (teaspoon) contains, as its active ingredient, a single dosage unit of the drug. Such dosage unit will contain about 30 mgs. of vinzolidine base as the 1,5-naphthalenedisulfonate salt. 1,5-Naphthalenedisulfonate salts of other indole-dihydroindole alkaloids represented by Formula (I) above can be formulated similarly.

Those skilled in the art will recognize the procedures used to prepare the formulations provided by the invention.

**Claims for the Contracting States: BE CH DE FR GB IT LI LU NL SE**

1. A 1,5-naphthalenedisulfonate salt of an indole-dihydroindole alkaloid of Formula (I)

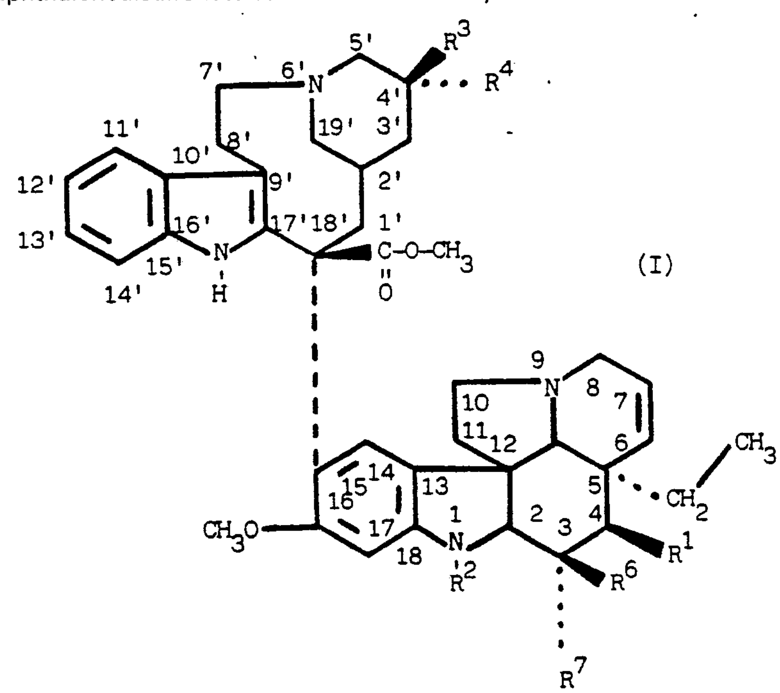

(I)

in which $R^1$ is OH or —$OCOCH_3$, $R^2$ is $CH_3$ or CHO; one of $R^3$ and $R^4$ is ethyl and the other is OH; when taken

singly $R^6$ is OH and $R^7$ is $COOCH_3$ or CO—$R^9$, in which $R^9$ is $NH_2$, NH—($C_1$—$C_3$ alkyl)-X, in which X is hydrogen, chloro or bromo, or carbo-($C_1$—$C_3$) alkoxy; or when $R^6$ and $R^7$ are taken together with the carbon atoms to which they are attached, they form a spiro-oxazolidenedione ring optionally substituted at $N_{3''}$ with $C_1$—$C_3$ alkyl or chloro-substituted $C_1$—$C_3$ alkyl.

2. A compound according to claim 1, said compound being a 1,5-naphthalenedisulfonate salt of vinzolidine.

3. Vincristine 1,5-naphthalenedisulfonate, vinblastine 1,5-naphthalenedisulfonate, or vindesine 1,5-naphthalenedisulfonate.

4. Vinzolidine 1,5-naphthalenedisulfonate.

5. A vinzolidine 1,5-naphthalenedisulfonate having the following X-ray diffraction pattern:

| d | $I/I_1$ | d | $I/I_1$ | d | $I/I_1$ |
|---|---|---|---|---|---|
| 12.15 | .80 | 4.57 | .36 | 2.89 | .04 |
| 10.88 | .16 | 4.42 | .44 | 2.77 | .04 |
| 9.80 | 1.00 | 4.17 | .40 | 2.67 | .04 |
| 8.14 | .16 | 3.97 | .32 | 2.54 | .04 |
| 7.58 | .28 | 3.76 | .64 | 2.46 | .04 |
| 7.24 | .20 | 3.56 | .40 | 2.40 | .04 |
| 6.52 | .64 | 3.34 | .44 | 2.28 | .04 |
| 6.18 | .16 | 3.26 | .24 | 2.22 | .04 |
| 5.90 | .04 | 3.08 | .04 | 2.15 | .04 |
| 5.55 | .16 | 2.95 | .04 | 2.08 | .06 |
| 4.89 | .44 | | | | |

6. A vinzolidine 1,5-naphthalenedisulfonate having the following X-ray diffraction pattern:

| d | $I/I_1$ | d | $I/I_1$ | d | $I/I_1$ |
|---|---|---|---|---|---|
| 12.65 | .14 | 4.36 | .28 | 2.91 | .03 |
| 10.54 | .48 | 4.16 | .14 | 2.79 | .03 |
| 9.47 | 1.00 | 4.07 | .14 | 2.68 | .03 |
| 7.98 | .07 | 3.92 | .07 | 2.60 | .02 |
| 7.32 | .17 | 3.78 | .14 | 2.49 | .03 |
| 7.15 | .59 | 3.57 | .07 | 2.38 | .02 |
| 6.69 | .21 | 3.47 | .28 | 2.32 | .02 |
| 6.11 | .17 | 3.34 | .17 | 2.25 | .03 |
| 5.44 | .41 | 3.23 | .07 | 2.22 | .02 |
| 5.14 | .31 | 3.13 | .07 | 2.17 | .02 |
| 4.71 | .52 | 3.03 | .03 | 2.12 | .02 |
| 4.50 | .10 | | | | |

7. A vinzolidine 1,5-naphthalenedisulfonate having the following X-ray diffraction pattern:

| d | $I/I_1$ | d | $I/I_1$ | d | $I/I_1$ |
|---|---|---|---|---|---|
| 10.85 | .55 | 5.40 | .20 | 3.42 | .15 |
| 10.16 | .05 | 5.07 | .10 | 3.30 | .05 |
| 9.26 | 1.00 | 4.75 | .40 | 3.15 | .08 |
| 7.41 | .05 | 4.33 | .30 | 3.02 | .05 |
| 6.76 | .55 | 4.20 | .15 | 2.92 | .08 |
| 6.28 | .80 | 4.00 | .20 | 2.82 | .05 |
| 5.68 | .20 | 3.75 | .05 | 2.69 | .05 |

8. A pharmaceutical formulation which comprises as an active ingredient, a compound of Formula (I), as defined in claim 1, associated with one or more pharmaceutically-acceptable carriers or vehicles therefor.

9. A pharmaceutical formulation which comprises as an active ingredient, vinzolidine 1,5-naphthalenedisulfonate, associated with one or more pharmaceutically-acceptable carriers or vehicles therefor.

10. A compound of Formula (I), as defined in claim 1, for use in cancer chemotherapy.

**Claims for the Contracting State: AT**

1. A process for preparing a 1,5-naphthalenedisulfonate salt of an indole-dihydroindole alkaloid of Formula (I)

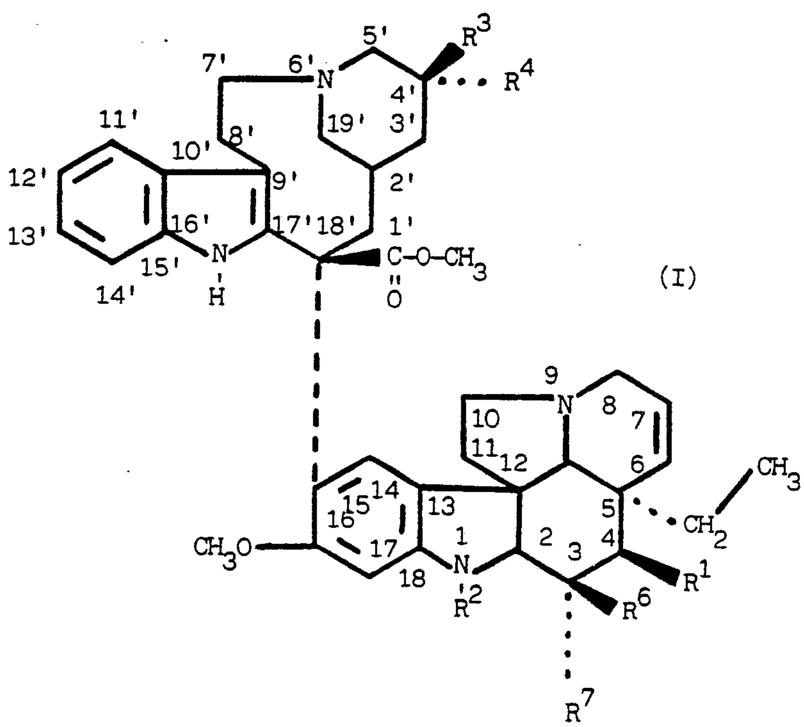

(I)

in which $R^1$ is OH or —$OCOCH_3$, $R^2$ is $CH_3$ or CHO; one of $R^3$ and $R^4$ is ethyl and the other is OH; when taken singly, $R^6$ is OH and $R^7$ is $COOCH_3$ or CO—$R^9$, in which $R^9$ is $NH_2$, NH—($C_1$—$C_3$ alkyl)-X, in which X is hydrogen, chloro or bromo, or carbo-($C_1$—$C_3$) alkoxy; or when $R^6$ and $R^7$ are taken together with the carbon atoms to which they are attached, they form a spiro-oxazolidenedione ring optionally substituted at $N_{3''}$ with $C_1$—$C_3$ alkyl or chloro-substituted $C_1$—$C_3$ alkyl, which comprises treating a compound of Formula (I), or a salt thereof, with 1,5-naphthalenedisulfonic acid, and recovering the product.

2. A process according to claim 1 for preparing a 1,5-naphthalenedisulfonate salt of vinzolidine.

3. A process as claimed in claim 1 for preparing vincristine 1,5-naphthalenedisulfonate, vinblastine 1,5-naphthalenedisulfonate, or vindesine 1,5-naphthalenedisulfonate.

4. A process according to claim 1 for preparing Vinzolidine 1,5-naphthalenedisulfonate.

5. A process according to claim 1 for preparing vinzolidine 1,5-naphthalenedisulfonate having the following X-ray diffraction pattern:

| d | $I/I_1$ | d | $I/I_1$ | d | $I/I_1$ |
|---|---|---|---|---|---|
| 12.15 | .80 | 4.57 | .36 | 2.89 | .04 |
| 10.88 | .16 | 4.42 | .44 | 2.77 | .04 |
| 9.80 | 1.00 | 4.17 | .40 | 2.67 | .04 |
| 8.14 | .16 | 3.97 | .32 | 2.54 | .04 |
| 7.58 | .28 | 3.76 | .64 | 2.46 | .04 |
| 7.24 | .20 | 3.56 | .40 | 2.40 | .04 |
| 6.52 | .64 | 3.34 | .44 | 2.28 | .04 |
| 6.18 | .16 | 3.26 | .24 | 2.22 | .04 |
| 5.90 | .04 | 3.08 | .04 | 2.15 | .04 |
| 5.55 | .16 | 2.95 | .04 | 2.08 | .06 |
| 4.89 | .44 | | | | |

6. A process as claimed in claim 1 for preparing vinzolidine 1,5-naphthalenedisulfonate having the following X-ray diffraction pattern:

| d | $I/I_1$ | d | $I/I_1$ | d | $I/I_1$ |
|---|---|---|---|---|---|
| 12.65 | .14 | 4.36 | .28 | 2.91 | .03 |
| 10.54 | .48 | 4.16 | .14 | 2.79 | .03 |
| 9.47 | 1.00 | 4.07 | .14 | 2.68 | .03 |
| 7.98 | .07 | 3.92 | .07 | 2.60 | .02 |
| 7.32 | .17 | 3.78 | .14 | 2.49 | .03 |
| 7.15 | .59 | 3.57 | .07 | 2.38 | .02 |
| 6.69 | .21 | 3.47 | .28 | 2.32 | .02 |
| 6.11 | .17 | 3.34 | .17 | 2.25 | .03 |
| 5.44 | .41 | 3.23 | .07 | 2.22 | .02 |
| 5.14 | .31 | 3.13 | .07 | 2.17 | .02 |
| 4.71 | .52 | 3.03 | .03 | 2.12 | .02 |
| 4.50 | .10 | | | | |

7. A process as claimed in claim 1 for preparing vinzolidine 1,5-naphthalenedisulfonate having the following X-ray diffraction pattern:

| d | I/I1 | d | I/I1 | d | I/I1 |
|---|---|---|---|---|---|
| 10.85 | .55 | 5.40 | .20 | 3.42 | .15 |
| 10.16 | .05 | 5.07 | .10 | 3.30 | .05 |
| 9.26 | 1.00 | 4.75 | .40 | 3.15 | .08 |
| 7.41 | .05 | 4.33 | .30 | 3.02 | .05 |
| 6.76 | .55 | 4.20 | .15 | 2.92 | .08 |
| 6.28 | .80 | 4.00 | .20 | 2.82 | .05 |
| 5.68 | .20 | 3.75 | .05 | 2.69 | .05 |

8. A compound of Formula (I), whenever prepared according to a process as claimed in claim 1.
9. A compound of Formula (I), as defined in claim 1, for use in cancer chemotherapy.

**Patentansprüche für die Vertragsstaaten: BE CH DE FR GB IT LI LU NL SE**

1. 1,5-Naphthalindisulfonatsalz eines Indol-Dihydroindol-Alkaloids der Formel (I)

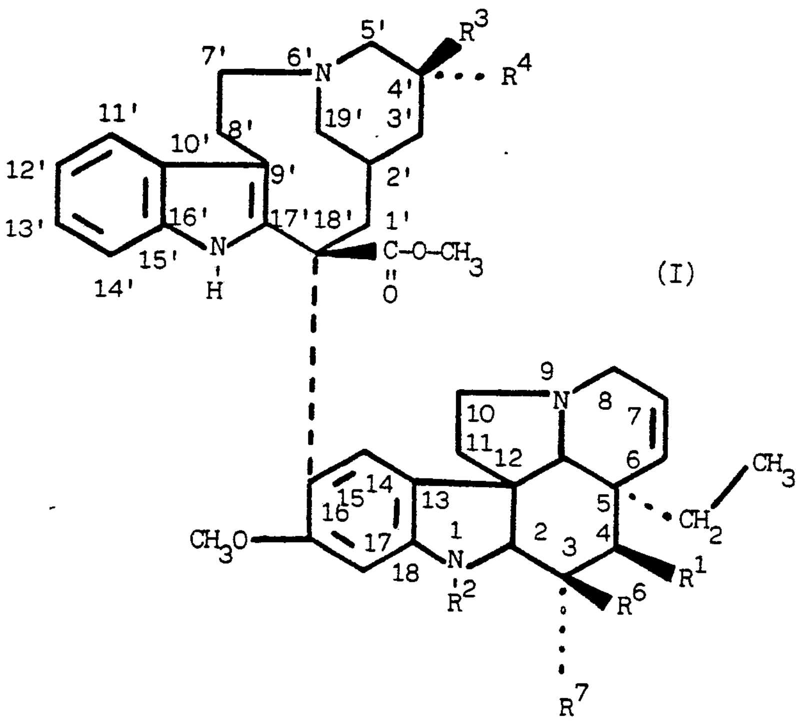

(I)

worin $R^1$ für OH oder —$OCOCH_3$ steht, $R^2$ für $CH_3$ oder CHO steht, einer der Substituenten $R^3$ oder $R^4$ Ethyl ist und der andere OH bedeutet und entweder einzeln $R^6$ für OH und $R^7$ für $COOCH_3$ oder CO—$R^9$ stehen, worin $R^9$ für $NH_2$, NH—($C_1$—$C_3$-Alkyl)-X, wobei X Wasserstoff, Chlor oder Brom ist, oder Carbo-($C_1$—$C_3$)-alkoxy steht, oder $R^6$ und $R^7$ zusammen mit den Kohlenstoffatomen, an die sie gebunden sind, einen spiro-Oxazolidindionring bilden, der am $N_{3''}$ gegebenenfalls durch $C_1$—$C_3$-Alkyl oder chlorsubstituiertes $C_1$—$C_3$-Alkyl substituiert ist.

2. Verbindung nach Anspruch 1, dadurch gekennzeichnet, daß es sich hierbei um ein 1,5-Naphthalindisulfonatsalz von Vinzolidin handelt.

3. Vincristin-1,5-naphthalindisulfonat, Vinblastin 1,5-naphthalindisulfonat oder Vindesin 1,5-naphthalindisulfonat.

4. Vinzolidin-1,5-naphthalindisulfonat.

5. Vinzolidin-1,5-naphthalindisulfonat, dadurch gekennzeichnet, daß es folgendes Röntgenbeugungsspektrum aufweist:

| d | $I/I_1$ | d | $I/I_1$ | d | $I/I_1$ |
|---|---|---|---|---|---|
| 12,15 | 0,80 | 4,57 | 0,36 | 2,89 | 0,04 |
| 10,88 | 0,16 | 4,42 | 0,44 | 2,77 | 0,04 |
| 9,80 | 1,00 | 4,17 | 0,40 | 2,67 | 0,04 |
| 8,14 | 0,16 | 3,97 | 0,32 | 2,54 | 0,04 |
| 7,58 | 0,28 | 3,76 | 0,64 | 2,46 | 0,04 |
| 7,24 | 0,20 | 3,56 | 0,40 | 2,40 | 0,04 |
| 6,52 | 0,64 | 3,34 | 0,44 | 2,28 | 0,04 |
| 6,18 | 0,16 | 3,26 | 0,24 | 2,22 | 0,04 |
| 5,90 | 0,04 | 3,08 | 0,04 | 2,15 | 0,04 |
| 5,55 | 0,16 | 2,95 | 0,04 | 2,08 | 0,06 |
| 4,89 | 0,44 | | | | |

6. Vinzolidin-1,5-naphthalindisulfonat, dadurch gekennzeichnet, daß es folgendes Röntgenbeugungsspektrum aufweist:

| d | $I/I_1$ | d | $I/I_1$ | d | $I/I_1$ |
|---|---|---|---|---|---|
| 12,65 | 0,14 | 4,36 | 0,28 | 2,91 | 0,03 |
| 10,54 | 0,48 | 4,16 | 0,14 | 2,79 | 0,03 |
| 9,47 | 1,00 | 4,07 | 0,14 | 2,68 | 0,03 |
| 7,98 | 0,07 | 3,92 | 0,07 | 2,60 | 0,02 |
| 7,32 | 0,17 | 3,78 | 0,14 | 2,49 | 0,03 |
| 7,15 | 0,59 | 3,57 | 0,07 | 2,38 | 0,02 |
| 6,69 | 0,21 | 3,47 | 0,28 | 2,32 | 0,02 |
| 6,11 | 0,17 | 3,34 | 0,17 | 2,25 | 0,03 |
| 5,44 | 0,41 | 3,23 | 0,07 | 2,22 | 0,02 |
| 5,14 | 0,31 | 3,13 | 0,07 | 2,17 | 0,02 |
| 4,71 | 0,52 | 3,03 | 0,03 | 2,12 | 0,02 |
| 4,50 | 0,10 | | | | |

7. Vinzolidin-1,5-naphthalindisulfonat, dadurch gekennzeichnet daß es folgendes Röntgenbeugungsspektrum aufweist:

| d | I/I$_1$ | d | I/I$_1$ | d | I/I$_1$ |
|---|---|---|---|---|---|
| 10,85 | 0,55 | 5,40 | 0,20 | 3,42 | 0,15 |
| 10,16 | 0,05 | 5,07 | 0,10 | 3,30 | 0,05 |
| 9,26 | 1,00 | 4,75 | 0,40 | 3,15 | 0,08 |
| 7,41 | 0,05 | 4,33 | 0,30 | 3,02 | 0,05 |
| 6,76 | 0,55 | 4,20 | 0,15 | 2,92 | 0,08 |
| 6,28 | 0,80 | 4,00 | 0,20 | 2,82 | 0,05 |
| 5,68 | 0,20 | 3,75 | 0,05 | 2,69 | 0,05 |

8. Pharmazeutische Formulierung, dadurch gekennzeichnet, daß sie eine Verbindung der Formel (I) gemäß Anspruch 1 als Wirkstoff in Kombination mit einem oder mehreren pharmazeutisch annehmbaren Trägern oder Hilfsstoffen hierfür enthält.

9. Pharmazeutische Formulierung, dadurch gekennzeichnet, daß sie Vinzolidin-1,5-naphthalindisulfonat als Wirkstoff in Kombination mit einem oder mehreren pharmazeutisch annehmbaren Trägern oder Hilfsstoffen hierfür enthält.

10. Verwendung einer Verbindung der Formel (I) nach Anspruch 1 zur chemotherapeutischen Behandlung von Krebs.

**Patentansprüche für den Vertragsstaat: AT**

1. Verfahren zur Herstellung eines 1,5-Naphthalindisulfonatsalzes eines Indol-Dihydroindol-Alkaloids der Formel (I)

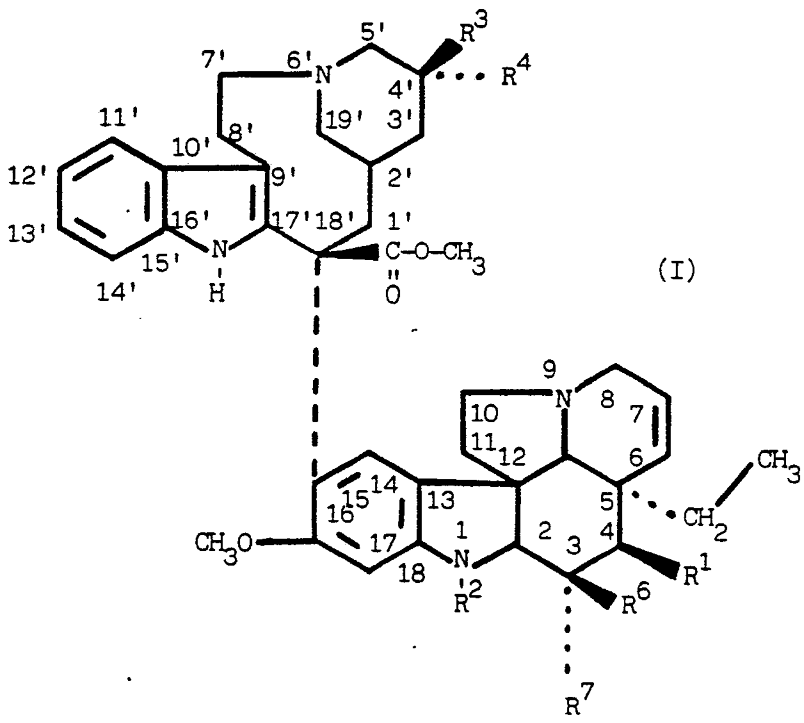

(I)

worin $R^1$ für OH oder —$OCOCH_3$ steht, $R^2$ für $CH_3$ oder CHO steht, einer der Substituenten $R^3$ oder $R^4$ Ethyl ist und der andere OH bedeutet und entweder einzeln $R^6$ für OH und $R^7$ für $COOCH_3$ oder CO—$R^9$ stehen, worin $R^9$ für $NH_2$, NH—($C_1$—$C_3$ Alkyl)-X, wobei X Wasserstoff, Chlor, oder Brom ist, oder Carbo-($C_1$—$C_3$)-alkoxy steht, oder $R^6$ und $R^7$ zusammen mit den Kohlenstoffatomen, an die sie gebunden sind, einen spiro-Oxazolidindionring bilden, der am $N_{3''}$ gegebenenfalls durch $C_1$—$C_3$-Alkyl oder chlorsubstituiertes $C_1$—$C_3$-

Alkyl substituiert ist, dadurch gekennzeichnet, daß man eine Verbindung der Formel (I) oder ein Salz hiervon mit 1,5-Naphthalindisulfonsäure behandelt und das Produkt gewinnt.

2. Verfahren nach Anspruch 1, dadurch gekennzeichnet, daß man ein 1,5-Naphthalindisulfonatsalz von Vinzolidin herstellt.

3. Verfahren nach Anspruch 1, dadurch gekennzeichnet, daß man Vincristin-1,5-Naphthalindisulfonat, Vinblastin-1,5-Naphthalindisulfonat oder Vindesin-1,5-Naphthalindisulfonat herstellt.

4. Verfahren nach Anspruch 1, dadurch gekennzeichnet, daß man Vinzolidin-1,5-naphthalindisulfonat herstellt.

5. Verfahren nach Anspruch 1, dadurch gekennzeichnet, daß man Vinzolidin-1,5-naphthalindisulfonat herstellt, das das folgende Röntgenbeugungsspektrum aufweist:

| d | $I/I_1$ | d | $I/I_1$ | d | $I/I_1$ |
|---|---|---|---|---|---|
| 12,15 | 0,80 | 4,57 | 0,36 | 2,89 | 0,04 |
| 10,88 | 0,16 | 4,42 | 0,44 | 2,77 | 0,04 |
| 9,80 | 1,00 | 4,17 | 0,40 | 2,67 | 0,04 |
| 8,14 | 0,16 | 3,97 | 0,32 | 2,54 | 0,04 |
| 7,58 | 0,28 | 3,76 | 0,64 | 2,46 | 0,04 |
| 7,24 | 0,20 | 3,56 | 0,40 | 2,40 | 0,04 |
| 6,52 | 0,64 | 3,34 | 0,44 | 2,28 | 0,04 |
| 6,18 | 0,16 | 3,26 | 0,24 | 2,22 | 0,04 |
| 5,90 | 0,04 | 3,08 | 0,04 | 2,15 | 0,04 |
| 5,55 | 0,16 | 2,95 | 0,04 | 2,08 | 0,06 |
| 4,89 | 0,44 | | | | |

6. Verfahren nach Anspruch 1, dadurch gekennzeichnet, daß man Vinzolidin-1,5-naphthalindisulfonat herstellt, das folgendes Röntgenbeugungsspektrum aufweist:

| d | $I/I_1$ | d | $I/I_1$ | d | $I/I_1$ |
|---|---|---|---|---|---|
| 12,65 | 0,14 | 4,36 | 0,28 | 2,91 | 0,03 |
| 10,54 | 0.48 | 4,16 | 0,14 | 2,79 | 0,03 |
| 9,47 | 1,00 | 4,07 | 0,14 | 2,68 | 0,03 |
| 7,98 | 0,07 | 3,92 | 0,07 | 2,60 | 0,02 |
| 7,32 | 0,17 | 3,78 | 0,14 | 2,49 | 0,03 |
| 7,15 | 0,59 | 3,57 | 0,07 | 2,38 | 0,02 |
| 6,69 | 0,21 | 3,47 | 0,28 | 2,32 | 0,02 |
| 6,11 | 0,17 | 3,34 | 0,17 | 2,25 | 0,03 |
| 5,44 | 0,41 | 3,23 | 0,07 | 2,22 | 0,02 |
| 5,14 | 0,31 | 3,13 | 0,07 | 2,17 | 0,02 |
| 4,71 | 0,52 | 3,03 | 0,03 | 2,12 | 0,02 |
| 4,50 | 0,10 | | | | |

7. Verfahren nach Anspruch 1, dadurch gekennzeichnet, daß man Vinzolidin-1,5-naphthalindisulfonat herstellt, das folgendes Röntgenbeugungsspektrum aufweist:

| d | I/I₁ | d | I/I₁ | d | I/I₁ |
|---|---|---|---|---|---|
| 10,85 | 0,55 | 5,40 | 0,20 | 3,42 | 0,15 |
| 10,16 | 0,05 | 5,07 | 0,10 | 3,30 | 0,05 |
| 9,26 | 1,00 | 4,75 | 0,40 | 3,15 | 0,08 |
| 7,41 | 0,05 | 4,33 | 0,30 | 3,02 | 0,05 |
| 6,76 | 0,55 | 4,20 | 0,15 | 2,92 | 0,08 |
| 6,28 | 0,80 | 4,00 | 0,20 | 2,82 | 0,05 |
| 5,68 | 0,20 | 3,75 | 0,05 | 2,69 | 0,05 |

8. Verbindung der Formel (I) dadurch gekennzeichnet, daß sie nach einem Verfahren gemäß Anspruch 1 hergestellt worden ist.

9. Verwendung einer Verbindung der Formel (I) nach Anspruch 1 zur chemotherapeutischen Behandlung von Krebs.

**Revendications pour les Etats contractants: BE CH DE FR GB IT LI LU NL SE**

1. Sel 1,5-naphtalène-disulfonate d'un alcaloïde d'indole-dihydroindole de formule (I):

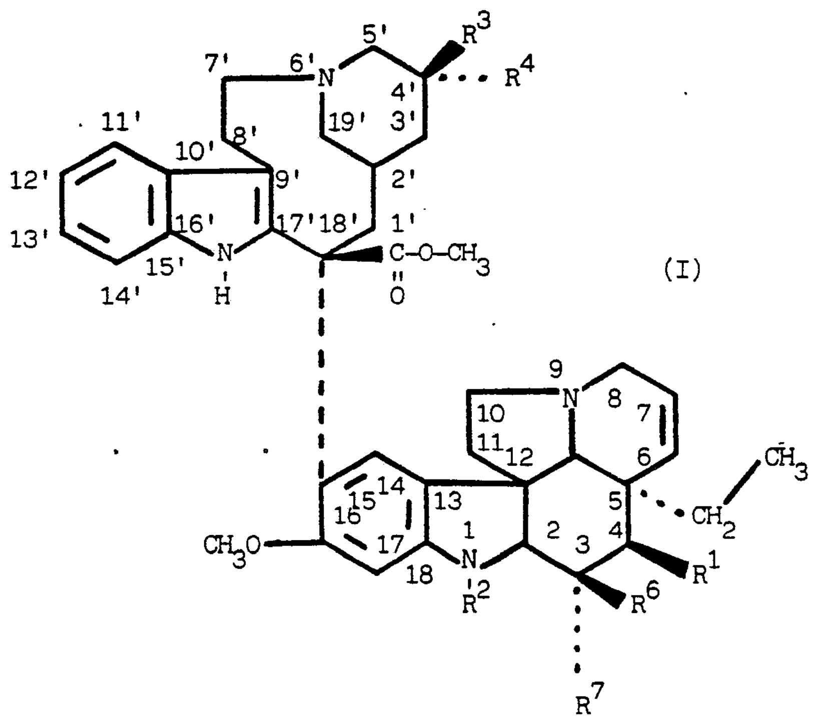

(I)

dans laquelle $R^1$ représente —OH ou —$OCOCH_3$, $R^2$ représente —$CH_3$ ou —CHO; un des radicaux $R^3$ et $R^4$ représente un groupe éthyle e l'autre, un groupe —OH; pris individuellement, $R^6$ représente —OH et $R^7$ représente —$COOCH_3$ ou —CO—$R^9$ ou $R^9$ représente —$NH_2$, NH-(alkyl en $C_1$—$C_3$)-X où X représente un atome d'hydrogène, un atome de chlore ou un atome de brome, ou encore un groupe carbo-alcoxy en $C_1$—$C_3$; ou lorsque $R^6$ et $R^7$ sont pris ensemble avec les atomes de carbone auxquels ils sont reliés, ils forment un noyau spiro-oxazolidine-dione éventuellement substitué en position $N_{3''}$ par un groupe alkyle en $C_1$—$C_3$ ou par groupe alkyle en $C_1$—$C_3$ substitué par un atome de chlore.

2. Composé selon la revendication 1, caractérisé en ce qu'il est un sel 1,5-naphtalène-disulfonate de vinzolidine.

3. 1,5-naphtalène-disulfonate de vincristine, 1,5-naphtalène-disulfonate de vinblastine ou 1,5-naphtalène-disulfonate de vindésine.

4. 1,5-naphtalène-disulfonate de vinzolidine.

5. 1,5-naphtalène-disulfonate de vinzolidine ayant le diagramme de diffraction des rayons X ci-après:

| d | $I/I_1$ | d | $I/I_1$ | d | $I/I_1$ |
|---|---|---|---|---|---|
| 12,15 | 0,80 | 4,57 | 0,36 | 2,89 | 0,04 |
| 10,88 | 0,16 | 4,42 | 0,44 | 2,77 | 0,04 |
| 9,80 | 1,00 | 4,17 | 0,40 | 2,67 | 0,04 |
| 8,14 | 0,16 | 3,97 | 0,32 | 2,54 | 0,04 |
| 7,58 | 0,28 | 3,76 | 0,64 | 2,46 | 0,04 |
| 7,24 | 0,20 | 3,56 | 0,40 | 2,40 | 0,04 |
| 6,52 | 0,64 | 3,34 | 0,44 | 2,28 | 0,04 |
| 6,18 | 0,16 | 3,26 | 0,24 | 2,22 | 0,04 |
| 5,90 | 0,04 | 3,08 | 0,04 | 2,15 | 0,04 |
| 5,55 | 0,16 | 2,95 | 0,04 | 2,08 | 0,06 |
| 4,89 | 0,44 | | | | |

6. 1,5-naphtalène-disulfonate de vinzolidine ayant le diagramme de diffraction des rayons X ci-après:

| d | $I/I_1$ | d | $I/I_1$ | d | $I/I_1$ |
|---|---|---|---|---|---|
| 12,65 | 0,14 | 4,36 | 0,28 | 2,91 | 0,03 |
| 10,54 | 0.48 | 4,16 | 0,14 | 2,79 | 0,03 |
| 9,47 | 1,00 | 4,07 | 0,14 | 2,68 | 0,03 |
| 7,98 | 0,07 | 3,92 | 0,07 | 2,60 | 0,02 |
| 7,32 | 0,17 | 3,78 | 0,14 | 2,49 | 0,03 |
| 7,15 | 0,59 | 3,57 | 0,07 | 2,38 | 0,02 |
| 6,69 | 0,21 | 3,47 | 0,28 | 2,32 | 0,02 |
| 6,11 | 0,17 | 3,34 | 0,17 | 2,25 | 0,03 |
| 5,44 | 0,41 | 3,23 | 0,07 | 2,22 | 0,02 |
| 5,14 | 0,31 | 3,13 | 0,07 | 2,17 | 0,02 |
| 4,71 | 0,52 | 3,03 | 0,03 | 2,12 | 0,02 |
| 4,50 | 0,10 | | | | |

7. 1,5-naphtalène-disulfonate de vinzolidine ayant le diagramme de diffraction des rayons X ci-après:

| d | I/I$_1$ | d | I/I$_1$ | d | I/I$_1$ |
|---|---|---|---|---|---|
| 10,85 | 0,55 | 5,40 | 0,20 | 3,42 | 0,15 |
| 10,16 | 0,05 | 5,07 | 0,10 | 3,30 | 0,05 |
| 9,26 | 1,00 | 4,75 | 0,40 | 3,15 | 0,08 |
| 7,41 | 0,05 | 4,33 | 0,30 | 3,02 | 0,05 |
| 6,76 | 0,55 | 4,20 | 0,15 | 2,92 | 0,08 |
| 6,28 | 0,80 | 4,00 | 0,20 | 2,82 | 0,05 |
| 5,68 | 0,20 | 3,75 | 0,05 | 2,69 | 0,05 |

8. Formulation pharmaceutique comprenant, comme ingrédient actif, un composé de formule (I) selon la revendication 1, en association avec un ou plusieurs supports ou véhicules pharmaceutiquement acceptables pour ce composé.

9. Formulation pharmaceutique comprenant, comme ingrédient actif, le 1,5-naphtalène-disulfonate de vinzolidine, en association avec un ou plusieurs supports ou véhicules pharmaceutiquement acceptables pour cet ingrédient.

10. Composé de formule (I) selon la revendication 1, en vue de l'utiliser dans le chimiothérapie du cancer.

**Revendications pour l'Etat contractant: AT**

1. Procédé de préparation d'un sel 1,5-naphtalène-disulfonate d'un alcaloïde-d'indole-dihydroindole de formule (I):

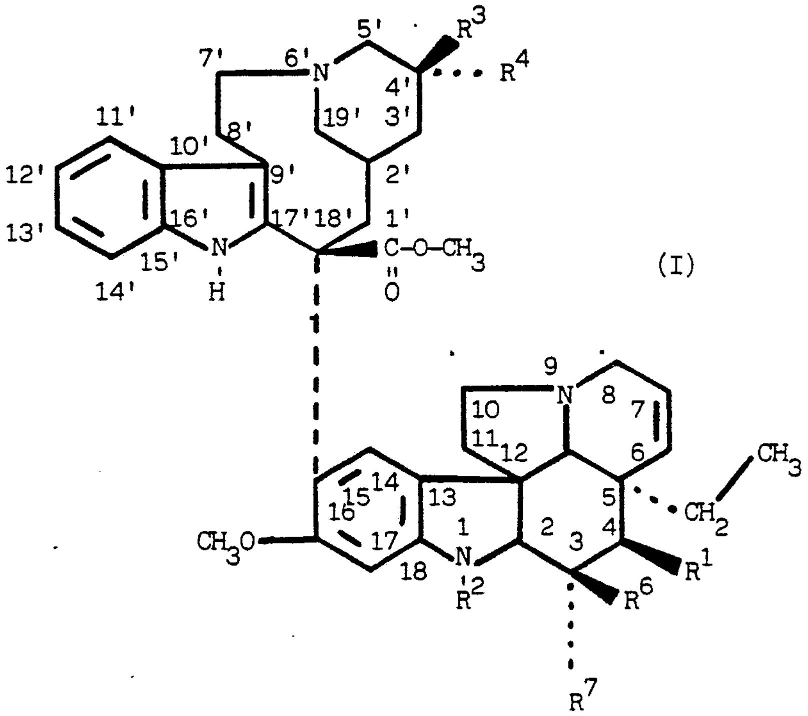

(I)

dans laquelle $R^1$ représente —OH ou —$OCOCH_3$, $R^2$ représente —$CH_3$ ou —CHO; un des radicaux $R^3$ et $R^4$ représente un groupe éthyle et l'autre, un groupe —OH; pris individuellement, $R^6$ représente —OH et $R^7$ représente —$COOCH_3$ ou —CO—$R^9$ où $R^9$ représente —$NH_2$, ou —NH-(alkyl en $C_1$—$C_3$)—X où X représente un atome d'hydrogène, un atome de chlore ou un atome de brome, ou un groupe carbo-alcoxy en $C_1$—$C_3$; ou, pris ensemble avec les atomes de carbone auxquels ils sont reliés, $R^6$ et $R^7$ forment un noyau spiro-oxazolidine-dione éventuellement substitué en position $N_{3''}$ avec un groupe alkyle en $C_1$—$C_3$ ou un groupe

alkyle en $C_1$—$C_3$ substitué par un atome de chlore, caractérisé en ce qu'il consiste à traiter un composé de formule (I) ou un de ses sels avec l'acide 1,5-naphthalène-disulfonique, puis récupérer le produit.

2. Procédé selon la revendication 1 en vue de préparer un sel 1,5-naphtalène-disulfonate de vinzolidine.

3. Procédé selon la revendication 1 en vue de préparer le 1,5-naphtalène-disulfonate de vincristine, le 1,5-naphtalène-disulfonate de vinblastine ou le 1,5-naphtalène-disulfonate de vindésine.

4. Procédé selon la revendication 1 en vue de préparer 1,5-naphtalène-disulfonate de vinzolidine.

5. Procédé selon la revendication 1 en vue de préparer 1,5-naphtalène-disulfonate de vinzolidine présentant le diagramme de diffraction des rayons X ci-après:

| d | $I/I_1$ | d | $I/I_1$ | d | $I/I_1$ |
|---|---|---|---|---|---|
| 12,15 | 0,80 | 4,57 | 0,36 | 2,89 | 0,04 |
| 10,88 | 0,16 | 4,42 | 0,44 | 2,77 | 0,04 |
| 9,80 | 1,00 | 4,17 | 0,40 | 2,67 | 0,04 |
| 8,14 | 0,16 | 3,97 | 0,32 | 2,54 | 0,04 |
| 7,58 | 0,28 | 3,76 | 0,64 | 2,46 | 0,04 |
| 7,24 | 0,20 | 3,56 | 0,40 | 2,40 | 0,04 |
| 6,52 | 0,64 | 3,34 | 0,44 | 2,28 | 0,04 |
| 6,18 | 0,16 | 3,26 | 0,24 | 2,22 | 0,04 |
| 5,90 | 0,04 | 3,08 | 0,04 | 2,15 | 0,04 |
| 5,55 | 0,16 | 2,95 | 0,04 | 2,08 | 0,06 |
| 4,89 | 0,44 | | | | |

6. Procédé selon la revendication 1 en vue de préparer le 1,5-naphtalène-disulfonate de vinzolidine présentant le diagramme de diffraction des rayons X ci-après:

| d | $I/I_1$ | d | $I/I_1$ | d | $I/I_1$ |
|---|---|---|---|---|---|
| 12,65 | 0,14 | 4,36 | 0,28 | 2,91 | 0,03 |
| 10,54 | 0.48 | 4,16 | 0,14 | 2,79 | 0,03 |
| 9,47 | 1,00 | 4,07 | 0,14 | 2,68 | 0,03 |
| 7,98 | 0,07 | 3,92 | 0,07 | 2,60 | 0,02 |
| 7,32 | 0,17 | 3,78 | 0,14 | 2,49 | 0,03 |
| 7,15 | 0,59 | 3,57 | 0,07 | 2,38 | 0,02 |
| 6,69 | 0,21 | 3,47 | 0,28 | 2,32 | 0,02 |
| 6,11 | 0,17 | 3,34 | 0,17 | 2,25 | 0,03 |
| 5,44 | 0,41 | 3,23 | 0,07 | 2,22 | 0,02 |
| 5,14 | 0,31 | 3,13 | 0,07 | 2,17 | 0,02 |
| 4,71 | 0,52 | 3,03 | 0,03 | 2,12 | 0,02 |
| 4,50 | 0,10 | | | | |

7. Procédé selon la revendication 1 en vue de préparer le 1,5-naphtalène-disulfonate de vinzolidine présentant le diagramme de diffraction des rayons X ci-après:

| d | $I/I_1$ | d | $I/I_1$ | d | $I/I_1$ |
|---|---|---|---|---|---|
| 10,85 | 0,55 | 5,40 | 0,20 | 3,42 | 0,15 |
| 10,16 | 0,05 | 5,07 | 0,10 | 3,30 | 0,05 |
| 9,26 | 1,00 | 4,75 | 0,40 | 3,15 | 0,08 |
| 7,41 | 0,05 | 4,33 | 0,30 | 3,02 | 0,05 |
| 6,76 | 0,55 | 4,20 | 0,15 | 2,92 | 0,08 |
| 6,28 | 0,80 | 4,00 | 0,20 | 2,82 | 0,05 |
| 5,68 | 0,20 | 3,75 | 0,05 | 2,69 | 0,05 |

8. Composé de formule (I), préparé par le procédé selon la revendication 1.

9. Composé de formule (I) tel que défini dans la revendication 1, en vue de l'utiliser dans la chimiothérapie du cancer.